# EUROPEAN PATENT APPLICATION

(11) **EP 3 949 913 A1**
(43) Date of publication of application: **09.02.2022**
(21) Application number: 20778610.4
(22) Date of filing: 25.03.2020
(51) Int. Cl.: A61F 2/844, A61L 31/16, A61L 31/14, A61F 2/958, A61B 17/00, A61F 2/00, A61K 47/69

(54) **FIBROSIS-INDUCING DRUG-ELUTING STENT FOR BLOCKING ELECTRIC CONDUCTION**

(30) Priority: 25.03.2019 KR 20190033733
(71) Applicant: Sungkwang Medical Foundation, Seoul 06135 (KR); Industry-Academic Cooperation Foundation, Yonsei University, Seoul 03722 (KR)
(72) Inventor: SUNG, Jung Hoon, Seongnam-si, Gyeonggi-do 13599 (KR); JUNG, Bo Young, Seoul 06713 (KR); YANG, Pil Sung, Seongnam-si, Gyeonggi-do 13505 (KR)
(74) Representative: Weickmann, Hans
(86) International application number: PCT/KR2020/004063
(87) International publication number: WO 2020/197262

(57) **Abstract**

The present invention provides a drug-eluting stent. The present invention includes a main structure, and a drug delivery layer that is applied to the main structure and includes a fibrosis drug, wherein, when the main structure comes into contact with a target tissue, the fibrosis drug of the drug delivery layer is released into the target tissue to perform fibrous of the target tissue.

## Description

### TECHNICAL FIELD

The present invention relates to a device, and more particularly, to a bioabsorbable drug-eluting stent that may treat tachycardia arrhythmia such as atrial fibrillation by eluting a fibrosis drug into a tissue to induce tissue fibrosis and blocking electrical conduction of the tissue therethrough.

### BACKGROUND ART

Tachycardia arrhythmia is caused by an erroneous signal that is generated in some tissues to stimulate the heart. For example, an electrical signal inducing atrial fibrillation is generated in a tissue of a pulmonary vein in the left atrium, and the electrical signal is transmitted to the heart to cause atrial fibrillation.

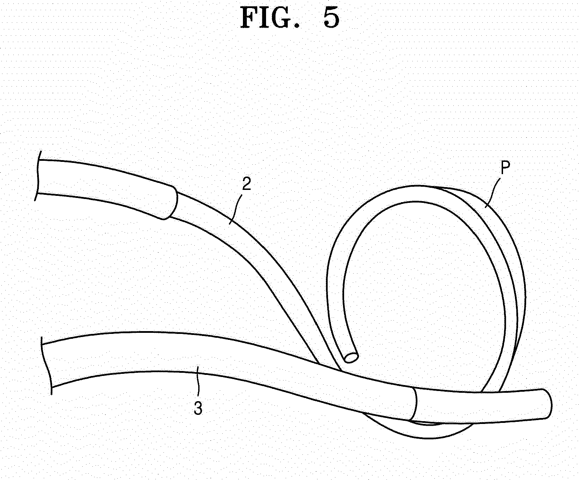

FIG. 5 is a view illustrating a radiofrequency catheter ablation used in the related art to treat atrial fibrillation. FIG. 5 illustrates a method of electrically separating a pulmonary vein from the left atrium by using the radiofrequency catheter ablation.

The radiofrequency catheter ablation is a method of destroying tissues around four pulmonary veins in the left atrium by using radio frequency (RF) energy. The radiofrequency catheter ablation is to destroy tissues by applying energy to a target tissue P that transmits an erroneous signal generated by a pulmonary vein to the left atrium.

In detail, when a pair of radiofrequency catheters 2 and 3 apply RF energy to the target tissue P between the left atrium and the pulmonary vein, the target tissue P is destroyed to electrically isolate the pulmonary vein from the left atrium. However, the radiofrequency catheter ablation may damage an organ such as the esophagus, and fatal complications such as stroke or pulmonary vein stenosis may occur after a procedure.

### DESCRIPTION OF EMBODIMENTS

### TECHNICAL PROBLEM

An object of the present invention is to provide a drug-eluting stent that may treat atrial fibrillation by performing fibrosis of a target tissue by using a released drug to block electrical conduction of the tissue and that is decomposed in a living body. However, these problems are examples, and the scope of the present invention is not limited thereto.

### SOLUTION TO PROBLEM

An aspect of the present invention provides a drug-eluting stent including a main structure, and a drug delivery layer that is applied to the main structure and includes a fibrosis drug, wherein, when the main structure comes into contact with a target tissue, the fibrosis drug of the drug delivery layer is released into the target tissue to perform fibrous of the target tissue.

### ADVANTAGEOUS EFFECTS OF DISCLOSURE

A drug-eluting stent according to an embodiment of the present invention may perform fibrosis of a target tissue to electrically separate the target tissue. A fibrotic target tissue may no longer transmit an electrical signal, and thus, atrial fibrillation due to transmission of an erroneous signal may be treated. In addition, the drug-eluting stent destroys only a target tissue, additional organ damage and complications may be prevented.

A drug-eluting stent according to an embodiment of the present invention is decomposed internally after performing fibrosis of a target tissue, and thus, there is no need for an additional procedure to remove the stent. When a fibrosis drug is released from a drug delivery layer, a main structure is also decomposed in a living body, and thus, an anatomical structure of the living body may be maintained.

A drug-eluting stent according to an embodiment of the present invention may simply and effectively release a fibrosis drug into a target tissue. When a main structure is expanded by inflation of a balloon, the main structure and a drug delivery layer come into contact with a target tissue, and thus, a fibrosis drug is directly released into the target tissue. In addition, in the drug-eluting stent, a cover layer may cover the drug delivery layer to control a drug elution speed or to increase biostability.

Of course, the scope of the present invention is not limited by the effects.

### BRIEF DESCRIPTION OF DRAWINGS

FIG. 1 is a view illustrating a state in which a drug-eluting stent according to an embodiment of the present invention is inserted.
FIG. 2A is a cross-sectional view of a main structure of FIG. 1.
FIG. 2B is a modified example of the main structure of FIG. 2.
FIG. 2C is another modified example of the main structure of FIG. 2.
FIGS. 3A to 3D are cross-sectional views illustrating a step of arranging a drug-eluting stent on a target tissue.
FIG. 4 is a flowchart illustrating a method of performing fibrosis of a target tissue using a drug-eluting stent.
FIG. 5 is a view illustrating a radiofrequency catheter ablation used in the related art to treat atrial fibrillation.
FIG. 6 is a photograph illustrating hematoxylin and eosin (H&E) stain of (a) normal venous vascular tissue without a drug-eluting stent and (b) fibrous oil-induced venous vascular tissue with a drug-eluting stent inserted therein.
FIG. 7 is a photograph illustrating Masson's Trichrome stain staining of (a) normal venous vascular tissue without a drug-eluting stent and (b) fibrous oil-induced venous vascular tissue with a drug-eluting stent.

### BEST MODE

An aspect of the present invention provides a drug-eluting stent including a main structure, and a drug delivery layer that is applied to the main structure and includes a fibrosis drug, wherein, when the main structure comes into contact with a target tissue, the fibrosis drug of the drug delivery layer is released into the target tissue to perform fibrous of the target tissue.

In addition, the main structure may have a mesh shape, and the drug may be released from the drug delivery layer to be decomposed and then may be decomposed in the target tissue.

In addition, the drug-eluting stent may further include a supporter extending in one direction to support the main structure.

In addition, the drug-eluting stent may further include a balloon that is inside the main structure to be contractible or inflatable.

In addition, the balloon in the main structure may expand to come into contact with the target tissue.

In addition, the fibrosis drug may be released into the target tissue to electrically isolate the target tissue.

In addition, the drug delivery layer may be formed by mixing the fibrosis drug with a polymer.

In addition, the drug-eluting stent may be installed between a pulmonary vein and a left atrium, and the fibrosis drug may perform fibrosis of a tissue between a pulmonary vein and a left atrium to block transmission of an electrical signal.

An aspect of the present invention provides a drug-eluting stent assembly including a catheter, a balloon mounted on one end of the catheter to be contractible or inflatable, a main structure that is outside the balloon and expanded when the balloon is inflated, and a drug delivery layer that is applied to the main structure and includes a fibrosis drug.

In addition, when the balloon is inflated, the main structure may come into contact with a target tissue, and the fibrosis drug of the drug delivery layer may be released into the target tissue to perform fibrosis of the target tissue.

In addition, the drug may be released from the drug delivery layer to the target tissue and then may be decomposed in the target tissue to electrically isolate the target tissue.

In addition, the drug-eluting stent may be installed between a pulmonary vein and a left atrium, and the fibrosis drug may perform fibrosis of a tissue between a pulmonary vein and a left atrium to block transmission of an electrical signal.

Other aspects, features, and advantages other than those described above will become apparent from the following detailed description, claims and drawings for implementing the following invention.

### MODE OF DISCLOSURE

Hereinafter, various embodiments of the present disclosure will be described with reference to the accompanying drawings. Various embodiments of the present disclosure may be modified in various ways and include various other embodiments, and specific embodiments are illustrated in the drawings and detailed descriptions thereof are provided. However, the present disclosure is not intended to limit the various embodiments to a specific embodiment and should be understood to include all changes and/or equivalents or substitutes included in the idea and scope of the various embodiments of the present disclosure. In connection with description of the drawings, similar reference numerals are used for similar components.

Expressions such as "include" or "may include" that may be used in various embodiments of the present disclosure indicate presence of corresponding disclosed function, operation, component, or so on, and do not limit one or more additional functions, operations, components, or so on. In addition, in various embodiments of the present disclosure, terms such as "include" and "have" are intended to designate existence of features, numbers, steps, operations, configuration elements, components, or a combination thereof described in the specification, and should be understood that a possibility of the existence or addition of one or more other features, numbers, steps, operations, configuration elements, components, or a combination thereof is not preliminarily excluded.

In various embodiments of the present disclosure, expressions such as "or" include any and all combinations of words listed together. For example, "A or B" may also include A, may also include B, or may include both A and B.

Expressions such as "first" or "second" used in various embodiments of the present disclosure may modify various configuration elements of various embodiments, but do not limit the configuration elements. For example, above expressions do not limit the order and/or importance of corresponding configuration elements. Above expressions may be used to distinguish one configuration element from another configuration element. For example, both the first user device and the second user device are user devices and indicate different user devices. For example, a first configuration element may be referred to as a second configuration element, and similarly, the second configuration element may also be referred to as the first configuration element without departing from the scope of the various embodiments of the present disclosure.

It should be understood that, when a configuration element is referred to as being "connected" or "coupled" to the other configuration element, the configuration element may be directly connected or coupled to the other configuration element, and there may be another new configuration element between the configuration element and the other configuration element. In addition, it should be understood that, when a configuration element is "directly connected" or "directly coupled" to another configuration element, there is no new configuration element between the configuration element and another configuration element.

Terms used in various embodiments of the present disclosure are used only to describe one specific embodiment and are not intended to limit the various embodiments of the present disclosure. Singular expressions include plural expressions unless the context clearly indicates otherwise.

Unless otherwise defined, all terms that include technical and scientific terms and are used herein have the same meaning as commonly understood by a person of ordinary skill in the art to which various embodiments of the present disclosure belong.

Terms as defined in a commonly used dictionary should be construed as having a meaning consistent with the meaning in the context of the related technology and are not construed in ideal or excessively formal meaning unless explicitly defined in various embodiments of the present disclosure.

Hereinafter, fibrosis is defined as a loss of function of a living tissue. The fibrosis may be understood as a loss of signaling function due to hardening of part of the living tissue. In addition, it may be understood that the signaling function is lost due to death of the living tissue.

Hereinafter, a fibrosis drug is defined as a drug that induces fibrosis of a living tissue. When a fibrosis drug is released into a target tissue P, the target tissue P becomes fibrotic to be electrically isolated from other tissues.

Hereinafter, the target tissue P is defined as a tissue in which a drug-eluting stent 100 is arranged and a fibrosis drug is released therefrom. In particular, the target tissue P is set around a pulmonary vein located in the left atrium, and electrical erroneous signals are generated in the target tissue P and transmitted therefrom, and thus, atrial fibrillation may occur.

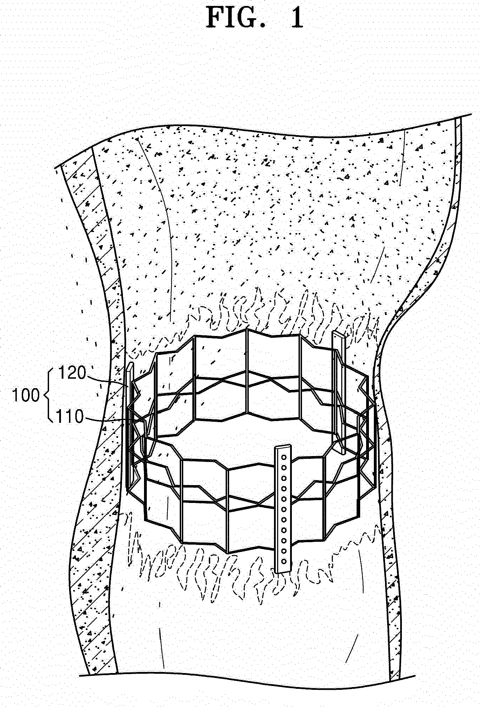

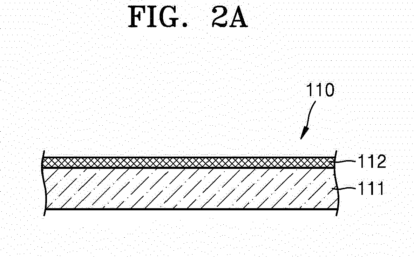

FIG. 1 is a view illustrating a state in which a drug-eluting stent 100 according to an embodiment of the present invention is inserted, and FIG. 2A is a cross-sectional view of a main structure 110 of FIG. 1.

Referring to FIGS. 1 and 2A, the drug-eluting stent 100 may include a main structure 110 and a supporter 120. The drug-eluting stent 100 may be inserted into a target tissue to perform fibrosis of the target tissue P.

In particular, the drug-eluting stent 100 may be used to treat atrial fibrillation. When a fibrosis drug of the drug-eluting stent 100 is released into the target tissue P to become fibrotic, an electrical erroneous signal generated by or transmitted from the target tissue may be prevented, and thus, atrial fibrillation may be prevented from occurring.

The main structure 110 may be inserted into the target tissue P, and a stent structure of the related art may be applied thereto. In one embodiment, the main structure 110 may be inserted into the target tissue P and support a wall of the target tissue P, and thus, a tissue may be formed into a shape set by the main structure 110 to perform fibrosis.

In another embodiment, the main structure 110 may be formed of a bioabsorbable material, that is, a bioabsorbable polymer. The main structure 110 may be formed of a polymer that may be decomposed and absorbed in a living body. At the same time, the main structure 110 may be formed of a biocompatible polymer.

For example, a bioabsorbable polymer may include poly-L-lactide (PLLA), poly-D-lactide (PDLA), polyglycolide (PGA), a copolymer of lactide and glycollide (PLGA), poly dioxanone, polygluconate, polylactic acid-polyethylene oxide copolymer, modified cellulose, collagen, poly (hydroxybutyrate), polyanhydride, polyphosphoester, poly (amino acid), or related copolymer, each of which has a characteristic decomposition speed in the body. For example, PGA and polydioxanone are relatively fast bioabsorbable materials (weeks to months), and PLLA and polycaprolactone are relatively slow bioabsorbable materials (months to years). Accordingly, an appropriate bioabsorbable material with an appropriate decomposition speed may be selected according to desired use of a drug-eluting stent.

The main structure 110 may have a mesh shape, and the whole may extend in one direction.

In addition, the main structure 110 may be elastically stretched. The main structure 110 may be inserted into a living body in a contracted state, and expand to maintain a shape when arranged in a target tissue.

The main structure 110 may include a base 111 and a drug delivery layer 112 applied to a surface of the base 111. The base 111 may be formed of a bioabsorbable, biodegradable, or biocompatible polymer as described above.

When a drug is released from the drug delivery layer 112 to be decomposed, the main structure 110 may be decomposed in the target tissue P thereafter. That is, when the drug is released from the drug delivery layer 112 and tissue fibrosis is performed, the main structure 110 may be decomposed in the living body to be removed.

The drug delivery layer 112 may be applied to at least a part of an outer circumferential surface or an inner circumferential surface of the main structure 110. The drug delivery layer 112 may be applied to one side of the base 111.

The drug delivery layer 112 includes a drug for fibrosis of a tissue. When the main structure 110 comes into contact with the drug delivery layer 112, fibrosis drug of the drug delivery layer 112 is released into the target tissue P, and thus, the target tissue P becomes fibrotic.

The fibrosis drug is not limited to a specific drug, and various drugs that are released by coming into contact with the tissue may be selected. When the fibrosis drug is released into the target tissue P, the target tissue P is electrically separated from other tissues of a living body. Thereby, the target tissue P is electrically and completely isolated in the living body.

In one embodiment, the fibrosis drug may include sodium tetradecyl sulfate. The sodium tetradecyl sulfate is a long-chain fatty acid with strong detergent properties. Accordingly, the sodium tetradecyl sulfate is an anionic detergent sclerosant that acts by disturbing the phospholipid membrane of cells.

In one embodiment, the sodium tetradecyl sulfate may have properties according to Formula 1 below. Sodium tetradecyl sulfate according to Formula 1 is water soluble and has unstable properties in a solid state and has physical properties that are transferred in an aqueous solution state.

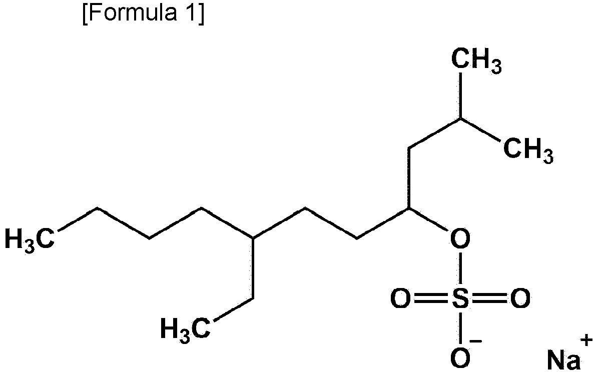

In another embodiment, the sodium tetradecyl sulfate may have properties according to Formula 2 below. The Sodium tetradecyl sulfate according to Formula 2 is water insoluble and is fine powder.

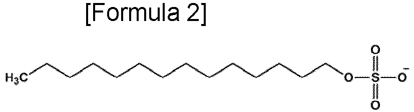

In another embodiment, a fibrosis drug may be obtained by mixing sodium tetradecyl sulfate and another drug. In this case, a ratio of the sodium tetradecyl sulfate may be set higher.

The drug delivery layer 112 may be formed by mixing a fibrosis drug with a polymer.

In one embodiment, the polymer may include poly lactic-co-glycolic acid (PLGA), and the fibrosis drug may include sodium tetradecyl sulfate. It may be prepared by dissolving a polymer and a fibrosis drug in a drug solution. Dimethyl sulfoxide (DMSO) may be used for the drug dissolution.

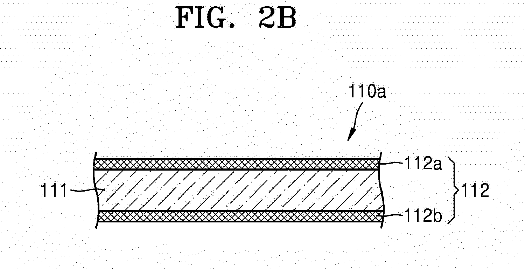

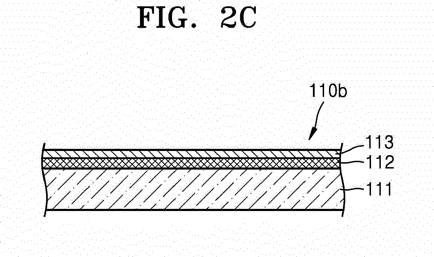

FIG. 2B is a modification example of the main structure of FIG. 2.

Referring to FIG. 2B, a main structure 110a includes a base 111 and a drug delivery layer 112. The drug delivery layer 112 may include a first drug layer 112a applied to an inner surface of the base 111 and a second drug layer 112b applied to the outside of the base 111.

Since a fibrosis drug is released from the first drug layer 112a and the second drug layer 112b, the target tissue P in contact with the main structure 110a may become rapidly fibrotic.

FIG. 2C is another modification example of the main structure of FIG. 2.

Referring to FIG. 2C, a main structure 110b may include a base 111, a drug delivery layer 112, and a cover layer 113. The drug delivery layer 112 is applied to one surface of the base 111, and the cover layer 113 is on one surface of the drug delivery layer 112. That is, the drug delivery layer 112 may be between the base 111 and the cover layer 113.

The cover layer 113 may maintain a contact between the target tissue P and the main structure 110b. Because the cover layer 113 is attached to the target tissue P, the main structure 110b may maintain a constant shape. The cover layer 113 may include an adhesive material with biostability.

In detail, the main structure 110b comes into contact with the target tissue P by inflation of a balloon 20. Thereafter, in order to prevent the balloon 20 from being contracted according to contraction of the main structure 110b, the cover layer 113 may maintain a contact between the target tissue P and the main structure 110b.

In addition, the cover layer 113 may cover the drug delivery layer 112 to ensure stability. A fibrosis drug included in the drug delivery layer 112 should not leak out of the target tissue. Until the main structure 110b reaches the target tissue P, the drug delivery layer 112 has to be safely sealed and there should be no contact with other tissues. Because the cover layer 113 covers the drug delivery layer 112, the fibrosis drug may be prevented from being released from the drug delivery layer 112.

In addition, the cover layer 113 may control a drug delivery speed of the drug delivery layer 112. The cover layer 113 is also formed of a biodegradable material. Accordingly, when the cover layer 113 is attached to the target tissue P, the cover layer 113 is preferentially decomposed, and then a fibrosis drug is released from the drug delivery layer 112 to the target tissue P. That is, the cover layer 113 may reduce the drug delivery speed of the drug delivery layer 112.

When the fibrosis drug is suddenly released into the target tissue P, tissue abnormality may occur. After the drug-eluting stent 100 is inserted into the target tissue P, the cover layer 113 forms an in-body adaptation period to ensure biostability.

In another embodiment, the cover layer 113 may include a drug that increases an absorption rate of a fibrosis drug. The cover layer 113 may include a drug that stabilizes a tissue. When a stabilizing drug is released from the cover layer 113 to the target tissue P, the target tissue P may be in a stabilization step capable of effectively absorbing a fibrosis drug. Thereafter, the fibrosis drug released from the drug delivery layer 112 may be released into the target tissue P at a rapid and high absorption rate.

In another embodiment, the drug delivery layer 112 may be applied only to a portion of the base 111. The drug delivery layer 112 may be applied only to a certain region of the base 111. In addition, the drug delivery layer 112 may be applied to be spaced apart in every section.

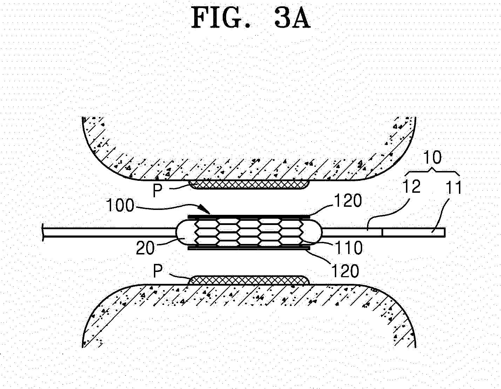

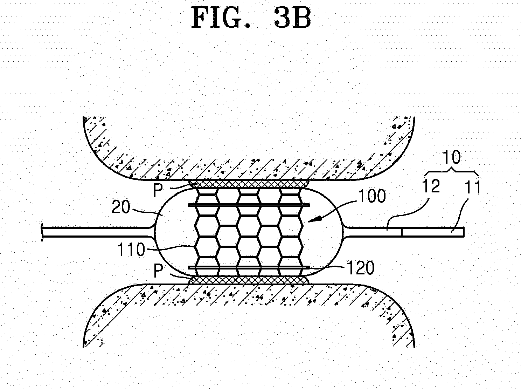

Referring back to FIG. 1, the supporter 120 may extend in one direction to support the main structure 110. Because the supporter 120 is connected to the main structure 110, the mesh-shaped main structure 110 may be aligned. The supporter 120 may support the main structure 110 to maintain the main structure 110 in a compressed state (refer to FIG. 3A). In addition, the supporter 120 may support the main structure 110 to maintain the main structure 110 in an expanded state (see FIG. 3B).

Like the main structure 110, the supporter 120 may also be formed of a bioabsorbable material, that is, a bioabsorbable polymer. The supporter 120 may be formed of a polymer that may be decomposed and absorbed in a living body. At the same time, the supporter 120 may be formed of a polymer with biocompatibility.

In another embodiment, the supporter 120 may store a drug. A fibrosis drug is stored in an inner space of the supporter 120, and after the supporter 120 is decomposed for a certain period of time, a large amount of fibrosis drug may be released.

In another embodiment, the drug delivery layer 112 may also be applied to a surface of the supporter 120. By increasing an area in contact with the drug delivery layer 112, a contact area through which the fibrosis drug is released may be increased.

In another embodiment, the supporter 120 may be omitted. That is, the drug-eluting stent 100 may be configured with only the main structure 110.

A drug-eluting stent assembly and the drug-eluting stent 100 may further include a catheter 10 and a balloon 20. Referring to FIG. 3A, the main structure 110 may be inserted into the balloon 20 arranged at an end portion of the catheter 10.

The catheter 10 may include a wire end portion 11 and a guide wire 12. The wire end portion 11 may be moved to the target tissue P, and the balloon 20 mounted on the guide wire 12 may be moved to the target tissue P.

The balloon 20 may be inside the main structure 110 and may be contracted or inflated. The main structure 110 may be outside the balloon 20 to be expanded when the balloon 20 is inflated. When the balloon 20 is inflated after moving to the target tissue P, the main structure 110 comes into contact with the target tissue P.

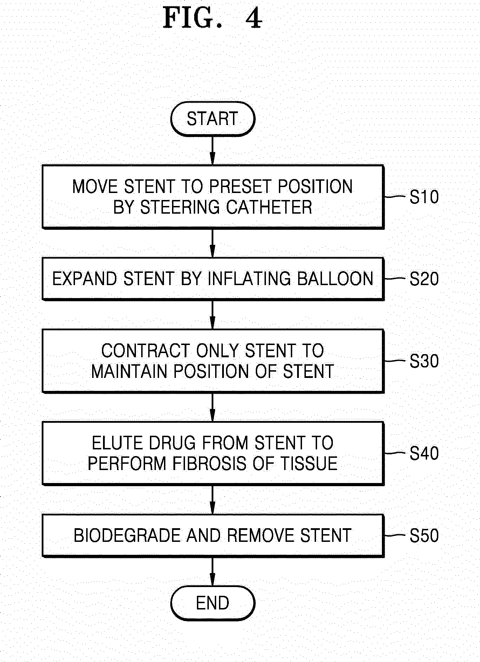

FIGS. 3A to 3D are cross-sectional views illustrating a step of arranging the drug-eluting stent 100 on a target tissue, and FIG. 4 is a flowchart illustrating a method of performing fibrosis of the target tissue by using the drug-eluting stent 100.

The method of performing fibrosis of the target tissue P by using the drug-eluting stent 100 includes a step of moving a stent to a preset position by steering a catheter (S10), a step of expanding the stent by inflating a balloon (S20), a step of contracting only the stent to maintain a position of the stent S30), a step of eluting a drug from the stent to perform fibrosis of a tissue (S40), and a step biodegrading and removing the stent (S50).

Referring to FIG. 3A, in the step of moving the stent to a preset position by steering the catheter (S10), a user steers the guide wire 12 to move the drug-eluting stent 100 to the target tissue P. At this time, the balloon 20 is installed in the catheter 10 in a contracted state, and the main structure 110 and the supporter 120 are connected to the balloon 20 to maintain the contracted state.

Referring to FIG. 3B, in the step of expanding the stent by inflating the balloon (S20), a user may inflate the balloon 20 to expand the drug-eluting stent 100. Because the main structure 110 may be elastically stretched, the main structure 110 may be expanded by the inflation of the balloon 20. Accordingly, the main structure 110 may come into contact with the target tissue P.

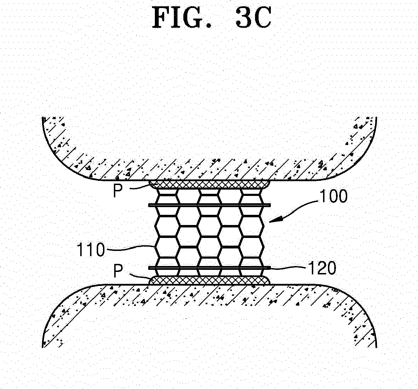

Referring to FIG. 3C, in the step S30 of contracting only the balloon to maintain a position of the stent, a user may contract the balloon 20 and remove the catheter 10 and the balloon 20. At this time, the main structure 110 of the drug-eluting stent 100 maintains an expanded shape, and thus, the main structure 110 is maintained in contact with the target tissue P.

In the step (S40) of eluting a drug from the stent to perform fibrosis of the tissue, a fibrosis drug is released from the drug delivery layer 112 and fibrosis F of the target tissue P is performed.

When the fibrosis drug is released into the target tissue P, the fibrosis F of the target tissue P is completed to be electrically isolated therefrom. There is no electrical signal connection between the target tissue P and other tissues. Accordingly, an electrical signal that is erroneously generated in or transmitted from the target tissue P is not transmitted to other tissues.

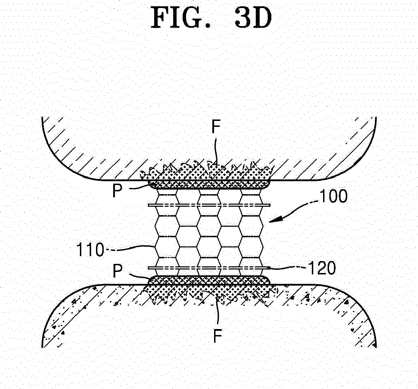

Referring to FIG. 3D, in the step S50 of biodegrading and removing the stent, the main structure 110 is decomposed and removed from a living body. A fibrosis drug is eluted from the drug delivery layer 112 or the main structure 110 is decomposed simultaneously with the elution of a drug. Accordingly, a user does not need to additionally remove the main structure 110.

The drug-eluting stent 100 may perform fibrosis of a target tissue to electrically isolate the target tissue. In addition, the drug-eluting stent 100 is decomposed in a living body, and thus, a user may safely perform an operation.

Atrial fibrillation is caused by an erroneous signal that is generated in some tissues to stimulate the heart. An electrical signal that induces atrial fibrillation is generated in a tissue of a pulmonary vein in the left atrium, and the electrical signal is transmitted to the heart to cause the atrial fibrillation.

FIG. 5 is a view illustrating a radiofrequency catheter ablation used in the related art to treat atrial fibrillation. FIG. 5 illustrates a method of separating a pulmonary vein from the left atrium by using the radiofrequency catheter ablation.

The radiofrequency catheter ablation is a method of destroying tissues around four pulmonary veins in the left atrium by using radio frequency (RF) energy. The radiofrequency catheter ablation is to destroy tissues by applying energy to the target tissue P where an erroneous signal is generated or transmitted.

In detail, when a pair of radiofrequency catheters 2 and 3 apply RF energy to the target tissue P between the left atrium and the pulmonary vein, the target tissue P is destroyed to electrically isolate the pulmonary vein from the left atrium. However, the radiofrequency catheter ablation may damage an organ such as the esophagus, and fatal complications such as stroke or pulmonary vein stenosis may occur after a procedure.

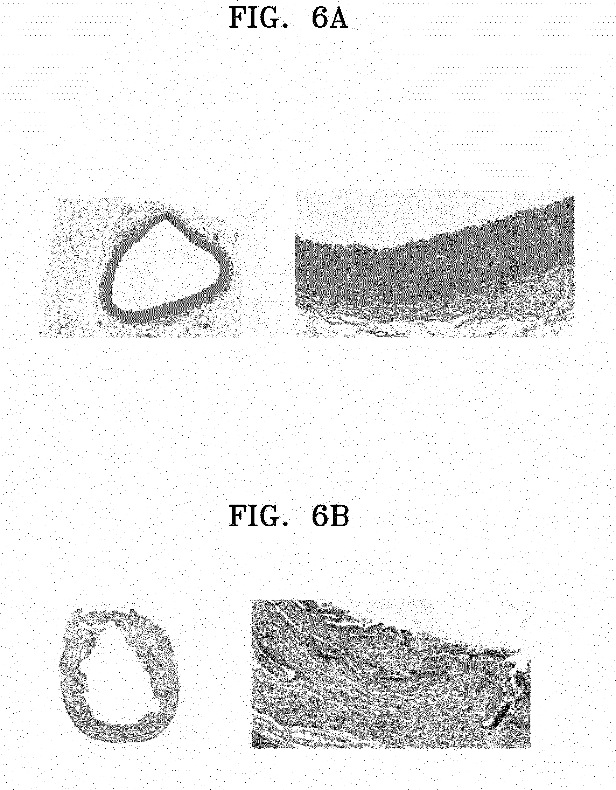

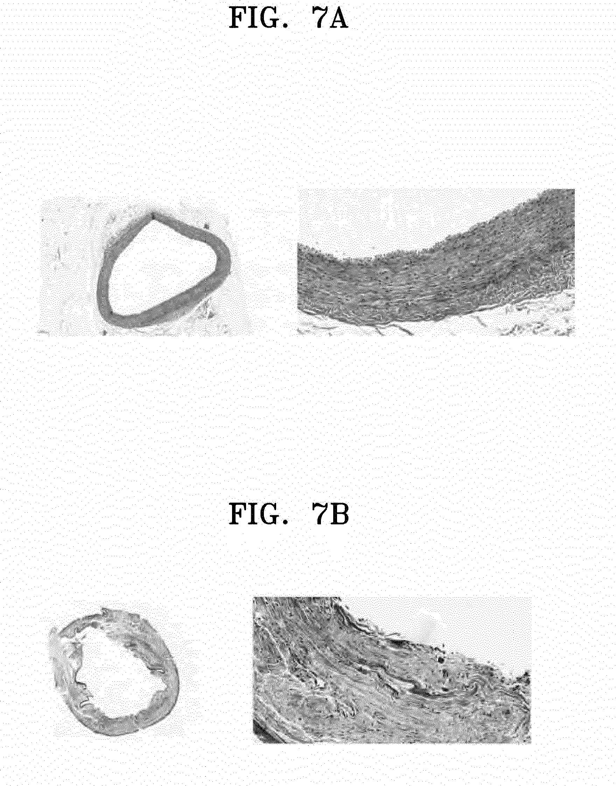

FIGS. 6 and 7 are photographs illustrating results of using the drug-eluting stent of FIG. 1. The experiment was performed by inserting a drug-eluting stent into an inferior vena cava (JVC) of a rabbit.
(a) of FIG. 6 is a photograph of hematoxylin and eosin (H&E) stain (red; tissue structure and blue; nucleus) in an inferior vena cava of a rabbit of a normal group, and (b) of FIG. 6 is a photograph of H&E stain in an inferior vena cava of a rabbit of an experimental group. (a) of FIG. 7 is a photograph of Masson's Trichrome stain (blue; collagen fiber) in an inferior vena cava of a rabbit of a normal group, and (b) of FIG. 7 is a photograph of Masson's Trichrome stain in an inferior vena cava of a rabbit of an experimental group.

A balloon dilatation stent coated with sodium tetradecyl sulfate was inserted through the inferior vena cava by using a rabbit. A stent with a diameter of 5 mm and a length of 8 mm was inserted (Ref: GSA-08-500, Genoss Co. (Gyeonggi-do, South Korea)). A breed of the rabbit was New Zealand White, and an average weight of the rabbit was 3.5 to 3.6 kg. After the stent was sacrificed four weeks after insertion, a tissue at a portion where the stent was inserted was obtained, and an average weight of the rabbit after four weeks was 3.8 to 3.9 kg.
(a) of FIG. 6 and (a) of FIG. 7 respectively illustrate hematoxylin staining (H&E stain) and Masson's trichrome stain for a blood vessel of a rabbit in a normal group, and (b) of FIG. 6 and (b) of FIG. 7 respectively illustrate H&E stain and Masson's trichrome stain for insertion of a drug-eluting stent into an inferior vena cava blood vessel of a rabbit in an experimental group.

In the normal group, a structure of a normal tissue, a position of a nucleus (H&E stain), a shape of collagen (Masson's trichrome stain; blue part), and so on may be confirmed. Meanwhile, compared to the normal group, a structure of a blood vessel was well maintained in the tissue into which the drug-eluting stent was inserted in the experimental group, but blood vessel tissue fibrosis (Masson's trichrome stain; blue part) was shown to be excellent. There was no stent thrombus in any of the inserted stents, and a side effect such as inflammation and a complication were not observed.

The drug-eluting stent 100 according to the present invention may perform fibrosis of the target tissue P to electrically separate the target tissue P. An electrical signal is no longer generated or transmitted in the fibrotic target tissue P, and thus, atrial fibrillation caused by an erroneous signal may be treated. In addition, the drug-eluting stent 100 destroys only the target tissue P, and thus, additional organ damage and a complication may be prevented.

In particular, a drug-eluting stent is installed between the pulmonary vein and the left atrium, and thus, a fibrosis drug may perform fibrosis of a tissue between the pulmonary vein and the left atrium to block transmission of an electrical signal. Thereby, atrial fibrillation or arrhythmia may be effectively treated. In detail, when the drug-eluting stent 100 is mounted on the pulmonary vein, a coated fibrosis-inducing drug is released into a pulmonary vein tissue for a certain period of time, thereby inducing fibrosis between the left atrium and a pulmonary vein and making a permanent electrical isolation, and thus, atrial fibrillation may be treated.

The drug-eluting stent 100 according to the present invention is decomposed internally after performing fibrosis of the target tissue P, and thus, there is no need for an additional procedure to remove the stent. When a fibrosis drug is released from the drug delivery layer 112, the main structure 110 is also decomposed in a living body, and thus, an anatomical structure of the living body may be maintained.

The drug-eluting stent 100 according to the present invention may simply and effectively release a fibrosis drug into the target tissue P. When the main structure 110 is expanded by inflation of the balloon 20, the main structure 110 and the drug delivery layer 112 come into contact with the target tissue P, and thus, a fibrosis drug is directly released into the target tissue P. In addition, in the drug-eluting stent 100, the cover layer 113 may cover the drug delivery layer 112 to control a drug elution speed or to increase biostability.

As such, the present invention is described with reference to the embodiments illustrated in the drawings, which are merely examples, and those skilled in the art will understand that various modifications and equivalent other embodiments are possible therefrom. Therefore, the true technical protection scope of the present invention should be determined by the technical idea of the appended claims.

### INDUSTRIAL APPLICABILITY

The present invention relates to a stent to be inserted into a living body. The present invention may be used for treatment of tachycardia arrhythmia such as atrial fibrillation by eluting a fibrosis drug into a tissue to induce tissue fibrosis and blocking electrical conduction of the tissue therethrough.

## Claims

1. A drug-eluting stent comprising:
a main structure; and
a drug delivery layer that is applied to the main structure and includes a fibrosis drug,
wherein, when the main structure comes into contact with a target tissue, the fibrosis drug of the drug delivery layer is released into the target tissue to perform fibrous of the target tissue.

2. The drug-eluting stent of claim 1, wherein the main structure has a mesh shape, and the drug is released from the drug delivery layer to be decomposed and then is decomposed in the target tissue.

3. The drug-eluting stent of claim 1, further comprising a supporter extending in one direction to support the main structure.

4. The drug-eluting stent of claim 1, further comprising a balloon that is inside the main structure to be contractible or inflatable.

5. The drug-eluting stent of claim 4, wherein the balloon in the main structure expands to come into contact with the target tissue.

6. The drug-eluting stent of claim 1, wherein the fibrosis drug is released into the target tissue to electrically isolate the target tissue.

7. The drug-eluting stent of claim 1, wherein the drug delivery layer is formed by mixing the fibrosis drug with a polymer.

8. The drug-eluting stent of claim 1, wherein the drug-eluting stent is installed between a pulmonary vein and a left atrium, and the fibrosis drug performs fibrosis of a tissue between a pulmonary vein and a left atrium to block transmission of an electrical signal.

9. A drug-eluting stent assembly comprising:
a catheter;
a balloon mounted on one end of the catheter to be contractible or inflatable;
a main structure that is outside the balloon and expanded when the balloon is inflated; and
a drug delivery layer that is applied to the main structure and includes a fibrosis drug.

10. The drug-eluting stent assembly of claim 9, wherein, when the balloon is inflated, the main structure comes into contact with a target tissue, and the fibrosis drug of the drug delivery layer is released into the target tissue to perform fibrosis of the target tissue.

11. The drug-eluting stent assembly of claim 9, wherein the drug is released from the drug delivery layer to the target tissue and then is decomposed in the target tissue to electrically isolate the target tissue.

12. The drug-eluting stent of claim 9, wherein the drug delivery layer is formed by mixing the fibrosis drug with a polymer.

13. The drug-eluting stent of claim 11, wherein the drug-eluting stent is installed between a pulmonary vein and a left atrium, and the fibrosis drug performs fibrosis of a tissue between a pulmonary vein and a left atrium to block transmission of an electrical signal.
